# FASCICULE DE BREVET EUROPEEN

(11) **EP 2 329 030 B1**
(45) Date de publication et mention de la délivrance du brevet: **26.10.2016**
(21) Numéro de dépôt: 09755941.3
(22) Date de dépôt: 24.09.2009
(51) Int. Cl.: C07C 407/00, C12P 7/16, C07C 409/04

(54) **FABRICATION D'HYDROPEROXYDE DE TERTIOBUTYLE A PARTIR DE MATIÈRES RENOUVELABLES**
HERSTELLUNG VON TERT.-BUTYLHYDROPEROXID AUS ERNEUERBAREN MATERIALIEN
MANUFACTURE OF TERTIOBUTYL HYDROPEROXIDE FROM RENEWABLE MATERIALS

(30) Priorité: 29.09.2008 FR 0856515
(43) Date de publication de la demande: 08.06.2011
(73) Titulaire: Arkema France, 92700 Colombes (FR)
(72) Inventeur: HUB, Serge, F-69100 Villeurbanne (FR); MAJ, Philippe, F-69530 Brignais (FR)
(74) Mandataire: Gavin, Pablo
(86) Numéro de dépôt international: PCT/FR2009/051808
(87) Numéro de publication internationale: WO 2010/034941

(56) Documents cités:
- EP-A- 0 501 577
- EP-A- 0 523 838
- EP-A- 1 953 129
- US-A- 4 863 862
- DATABASE CAPLUS [Online] CHEMICAL ABSTRACTS SERVICE, COLUMBUS, OHIO, US; 1972, CHIZHOV ET AL.: "Reaction of isobutylene with hydrogen peroxide and sulfuric acid" XP002532997 Database accession no. 1972:71946 & ZHURNAL PRIKLADNOI KHIMII (SANKT-PETERSBURG, RUSSIAN FEDERATION), vol. 44, no. 12, 1971, pages 2692-2696,
- DATABASE CAPLUS [Online] CHEMICAL ABSTRACTS SERVICE, COLUMBUS, OHIO, US; 1961, MESHCHERYAKOV A.P.: "Synthesis of peroxides based on isobutylene" XP002532998 Database accession no. 1961:117987 & SBORNIK NAUCH. RABOT, AKAD. NAUK BELORUS. S.S.R., INST. FIZ.-ORG. KHIM., no. 8, 1960, pages 3-12,
- FUJII T ET AL: "The role of l-phenylalanine in the production of isobutene by Rhodotorula minuta" JOURNAL OF FERMENTATION AND BIOENGINEERING, SOCIETY OF FERMENTATION TECHNOLOGY, JP, vol. 67, no. 2, 1 janvier 1989 (1989-01-01), pages 115-118, XP025737270 ISSN: 0922-338X [extrait le 1989-01-01]

## Description

La présente invention se rapporte à un procédé de fabrication d'hydroperoxyde de tertiobutyle à partir de matières premières renouvelables.

En particulier, l'invention se rapporte à un procédé de fabrication d'hydroperoxyde de tertiobutyle à partir d'alcools issus de la fermentation de matières premières renouvelables, de préférence les matières premières renouvelables sont des matières végétales.

L'hydroperoxyde de tertiobutyle (TBHP) encore appelé 1,1 diméthylhydroperoxide ou 2-Hydroperoxy 2-Methylpropane correspond à la formule :

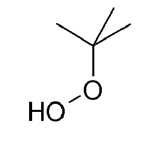

L'hydroperoxyde de tertiobutyle est connu pour son utilisation en tant qu'initiateur de polymérisation : dans des conditions opératoires (température, conditions rédox...) appropriées, il se décompose pour produire des radicaux libres qui vont générer des sites actifs sur le squelette du composé à polymériser.

Il peut aussi servir comme matière première pour la synthèse d'autres peroxydes organiques comme les peresters, peroxyacétals ou peracétals, dialkylperoxydes, monoperoxypercarbonates.

Il existe plusieurs voies de synthèse de l'hydroperoxyde de tertiobutyle à partir de l'isobutène, de l'isobutane ou du tertio-butanol, ces matières premières étant toutes obtenues à partir de matières premières d'origine fossile (pétrole) non renouvelables.

Or les ressources en pétrole sont limitées, l'extraction du pétrole requiert d'aller creuser de plus en plus profond et dans des conditions techniques toujours plus difficiles nécessitant des équipements sophistiqués et la mise en oeuvre de procédés toujours plus coûteux en énergie. Ces contraintes ont une conséquence directe sur le coût de fabrication de l'hydroperoxyde de tertiobutyle .

Le problème à l'origine de la présente demande de brevet est de proposer d'autres voies de synthèse de l'hydroperoxyde de tertiobutyle.

De manière avantageuse et surprenante, les inventeurs de la présente invention ont mis en oeuvre un procédé de fabrication industriel de l'hydroperoxyde de tertiobutyle à partir de matières premières renouvelables.

Le procédé selon l'invention permet de s'affranchir au moins en partie des matières premières d'origine fossile et de les remplacer par des matières premières renouvelables.

L'hydroperoxyde de tertiobutyle obtenu suivant le procédé selon l'invention est de qualité telle qu'il peut d'être utilisé dans toutes les applications dans lesquelles il est connu d'utiliser l'hydroperoxyde de tertiobutyle , même les applications les plus exigeantes.

L'invention a pour objet un procédé de fabrication de l'hydroperoxyde de tertiobutyle comprenant les étapes suivantes :
a) fermentation de matières premières renouvelables et, éventuellement purification pour produire un mélange comprenant au moins du butanol ;
b) déshydratation du butanol en butène;
c) conversion du butène en isobutène,
d) réaction avec du peroxyde d'hydrogène de manière à produire de l'hydroperoxyde de tertiobutyle,
e) isolation de l'hydroperoxyde de tertiobutyle.

L'étape c) peut aussi être suivie d'une étape d'hydratation de l'isobutène en tertiobutanol, selon cette variante, à l'étape d) le tertiobutanol réagit avec le peroxyde d'hydrogène.

D'autres objets, aspects, caractéristiques de l'invention apparaîtront à la lecture de la description suivante.

L'étape a) du procédé de fabrication de l'hydroperoxyde de tertiobutyle selon l'invention comprend la fermentation de matières premières renouvelables pour produire un mélange comprenant au moins du butanol.

Une matière première renouvelable est une ressource naturelle, par exemple animale ou végétale, dont le stock peut se reconstituer sur une période courte à l'échelle humaine. Il faut en particulier que ce stock puisse se renouveler aussi vite qu'il est consommé. Par exemple, les matières végétales présentent l'avantage de pouvoir être cultivées sans que leur consommation aboutisse à une diminution apparente des ressources naturelles.

A la différence des matériaux issus de matières fossiles, les matières premières renouvelables contiennent du ¹⁴C. Tous les échantillons de carbone tirés d'organismes vivants (animaux ou végétaux) sont en fait un mélange de 3 isotopes : ¹²C (représentant environ 98,892 %), ¹³C (environ 1,108 %) et ¹⁴C (traces: 1,2.10⁻¹⁰ %). Le rapport ¹⁴C/¹²C des tissus vivants est identique à celui de l'atmosphère. Dans l'environnement, le ¹⁴C existe sous deux formes prépondérantes : sous forme de gaz carbonique (CO₂), et sous forme organique, c'est-à-dire de carbone intégré dans des molécules organiques.

Dans un organisme vivant, le rapport ¹⁴C/¹²C est maintenu constant par le métabolisme car le carbone est continuellement échangé avec l'environnement extérieur. La proportion de ¹⁴C étant constante dans l'atmosphère, il en est de même dans l'organisme, tant qu'il est vivant, puisqu'il absorbe ce ¹⁴C au même titre que le ¹²C ambiant. Le rapport moyen de ¹⁴C/¹²C est égal à 1,2x10⁻¹².

Le ¹²C est stable, c'est-à-dire que le nombre d'atomes de ¹²C dans un échantillon donné est constant au cours du temps. Le ¹⁴C est radioactif, le nombre d'atomes de ¹⁴C dans un échantillon décroît au cours du temps (t), sa demi-vie étant égale à 5730 ans.

La teneur en ¹⁴C est sensiblement constante depuis l'extraction des matières premières renouvelables, jusqu'à la fabrication de l'hydroperoxyde de tertiobutyle selon le procédé de l'invention et même jusqu'à la fin de l'utilisation dudit hydroperoxyde de tertiobutyle .

Par conséquent, la présence de ¹⁴C dans un matériau, et ce, quelle qu'en en soit la quantité, donne une indication sur l'origine des molécules le constituant, à savoir qu'elles proviennent de matières premières renouvelables et non de matériaux fossiles.

La quantité de ¹⁴C dans un matériau peut être déterminée par l'une des méthodes décrites dans la norme ASTM D6866-06 (Standard Test Methods for Determining the Biobased Content of Natural Range Materials Using Radiocarbon and Isotope Ratio Mass Spectrometry Analysis).

Cette norme comporte trois méthodes de mesure du carbone organique issu de matières premières renouvelables, dénommé en langue anglaise « biobased carbon ». Les proportions indiquées pour l'hydroperoxyde de tertiobutyle obtenu selon le procédé de l'invention sont de préférence mesurées selon la méthode par spectrométrie de masse ou la méthode par spectrométrie à scintillation liquide décrite dans cette norme et tout préférentiellement par spectrométrie de masse.

Ces méthodes de mesure évaluent le rapport des isotopes ¹⁴C/¹²C dans l'échantillon et le comparent à un rapport des isotopes ¹⁴C/¹²C dans un matériau d'origine biologique donnant le 100% standard, afin de mesurer le pourcentage de carbone organique de l'échantillon.

De préférence, l'hydroperoxyde de tertiobutyle obtenu selon le procédé de l'invention comprend une quantité de carbone issu de matières premières renouvelables supérieure à 20%, de préférence supérieure à 50% en masse par rapport à la masse totale de carbone d'hydroperoxyde de tertiobutyle.

En d'autres termes, l'hydroperoxyde de tertiobutyle peut comporter au moins 0,24 10⁻¹⁰ % en masse de ¹⁴C, et de préférence au moins 0,6 10⁻¹⁰ % en masse ¹⁴C.

Avantageusement, la quantité de carbone issu de matières premières renouvelables supérieure à 60%, de préférence supérieure à 70%, de manière encore plus préférée 80%.

En tant que matières premières renouvelables, on pourra utiliser des matières végétales, des matières d'origine animale ou des matières d'origine végétale ou animale issues de matériaux récupérés (matériaux recyclés).

A titre de matières végétales, on compte notamment les sucres, les amidons ainsi que toute matière végétale contenant au moins des sucres et/ou amidons.

Les matières végétales contenant des sucres sont essentiellement la canne à sucre et la betterave sucrière, on peut également citer l'érable, le palmier-dattier, le palmier à sucre, le sorgho, l'agave américain ; les matières végétales contenant des amidons sont essentiellement les céréales et légumineuses comme le maïs, le blé, l'orge, le sorgho, le seigle, le froment, le riz, la pomme de terre, le manioc, la patate douce, ou encore les algues.

Parmi les matières issues de matériaux récupérés, on peut notamment citer les déchets végétaux ou organiques comprenant des sucres et/ou amidons.

Avantageusement, des matières premières de basse qualité pourront être utilisées comme par exemple des pommes de terre qui auraient gelé, des céréales contaminées par des mycotoxines ou encore des excédents de betteraves sucrières.

De préférence les matières premières renouvelables sont des matières végétales.

En tant que matières premières renouvelables, on peut également utiliser de la cellulose ou hémicellulose voire la lignine qui, en présence des microorganismes adéquats, peuvent être transformées en matières comprenant du sucre. Parmi ces matières renouvelables, on compte la paille, le bois, le papier, qui peuvent provenir avantageusement de matériaux récupérés.

La fermentation des matières renouvelables s'effectue en présence d'un ou plusieurs microorganismes adéquats, ce microorganisme peut éventuellement avoir été modifié naturellement par une contrainte chimique ou physique, ou génétiquement on parle alors de mutant. Classiquement le microorganisme utilisé est un *Clostridium,* avantageusement il s'agira du *Clostridium acetobutylicum* ou d'un de ses mutants.

Les listes présentées ci-dessus ne sont pas limitatives.

L'étape de fermentation peut également être précédée d'une étape d'hydrolyse des matières premières au moyen d'une enzyme de type cellulase ou d'un complexe de plusieurs enzyme de type cellulase.

La fermentation conduit généralement à la production d'un mélange de produits, typiquement la production de butanol s'accompagne d'une production d'acétone.

Ainsi, avantageusement, l'étape de fermentation est suivie d'une étape d'isolation du butanol.

Le butanol est généralement essentiellement constitué de 1-butanol.

L'isolation du butanol consiste généralement en une séparation des différents produits de la réaction par exemple par distillation hétéroazéotropique. Cette séparation peut également être suivie d'une distillation destinée à obtenir le butanol sous forme plus concentrée.

Un autre avantage du procédé selon l'invention est son économie en énergie : l'étape de fermentation et l'éventuelle étape d'hydrolyse du procédé selon l'invention sont effectuées à des températures faibles. Leur coût énergétique est aussi faible en comparaison au coût d'extraction du butane ou du benzène.

Cette économie d'énergie s'accompagne également d'une diminution du taux de CO₂ émis dans l'atmosphère.

A l'étape b) est mise en oeuvre la déshydratation du butanol en butène, cette réaction s'effectue en présence d'un acide fort tel que par exemple l'acide sulfurique (H₂SO₄) ou phosphorique à une température d'environ 120°C.

Lors de l'étape c), le butène obtenu à l'étape b) est converti en isobutène.

Cette réaction est mise en oeuvre par mise en contact du butène avec un tectométallosilicate ayant une structure cristalline de ferriérite, à une température comprise entre 150 °C et 450 °C, une pression partielle du butène de 0,5 bar et une pression totale comprise entre 0,5 et 25 bar.

A titre de tectométallosilicates ayant une structure cristalline de ferriérite utilisables à l'étape c), on compte les ferriérites suivantes FU-9, ISI-6, Nu-23, ZSM-21, ZSM-35 et ZSM-38. Les tectométallosilicates utilisables à l'étape c) contiennent du silicium et au moins un élément du groupe formé par le fer, le gallium, et l'aluminium de préférence ils contiennent du silicium et de l'aluminium. De manière préférée, les tectométallosilicates sont sous forme hydrogène.

Lors de l'étape d), l'isobutène obtenu à l'étape c) réagit avec du peroxyde d'hydrogène (H₂O₂) de manière à produire de l'hydroperoxyde de tertiobutyle..

Cette réaction s'effectue en phase liquide, en présence d'acide sulfurique, sous agitation continue et vigoureuse. De préférence la température de réaction est comprise entre 30°C et 75°C à pression atmosphérique.

L'étape c) peut aussi être suivie d'une étape d'hydratation de l'isobutène en tertiobutanol, selon cette variante à l'étape d) c'est le tertiobutanol qui réagit avec le peroxyde d'hydrogène. L'hydratation de l'isobutène en tertiobutanol est obtenue en présence d'acide sulfurique dilué (50 à 65 % poids), soit en présence de résines échangeuses d'ions acides. La réaction du tertiobutanol avec le peroxyde d'hydrogène pour donner l'hydroperoxyde de tertiobutyle s'effectue dans les mêmes conditions que l'étape d) décrite ci-dessus.

L'étape e) du procédé concerne l'isolation de l'hydroperoxyde de tertiobutyle obtenu à l'issue de l'étape d).

Un avantage supplémentaire du procédé mis en oeuvre dans la présente demande concerne les impuretés présentes dans l'isobutène utilisé à l'étape d).

Selon une voie classique l'isobutène est obtenu par craquage et vapocraquage de naphta (issu du pétrole). Ces techniques, en particulier le vapocraquage, permettent d'obtenir une coupe C₄ comprenant de l'iso-butène mais également des composés dioléfiniques et des composés acétyléniques tels que les propyne, vinylacétylène, butyne-1 et butyne -2.

Ces composés dioléfiniques et acétyléniques sont susceptibles d'initier des réactions de polymérisation, de contribuer à la formation de gommes, d'inhiber les sites actifs des catalyseurs, d'influer sur les performances des solvants, il est donc nécessaire de les séparer de l'isobutène.

L'isobutène obtenu à l'étape c) du procédé selon la présente invention présente l'avantage de ne pas comprendre de tels composés acétyléniques, le procédé selon l'invention ne requiert dons pas la mis en oeuvre de cette étape de séparation qui peut s'avérer difficile et coûteuse.

L'hydroperoxyde de tertiobutyle obtenu à partir de matières d'origine renouvelable peut être utilisé pour la fabrication de peresters, de peroxyacétals ou peracétals de dialkylperoxydes, de monoperoxypercarbonates. Ces peresters, peroxyacétals ou peracétals dialkylperoxydes, de monoperoxypercarbonates sont notamment utilisables en tant qu'initiateurs de polymérisation

Dans la fabrication de peresters, on peut citer l'utilisation de l'hydroperoxyde de tertiobutyle obtenu à partir de matières d'origine renouvelable avec :
▪ le chlorure de néodécanoyle pour former le tert-butyl peroxynéodécanoate, ce perester est par exemple commercialisé sous le nom LUPEROX 10 par la société Arkema ;
▪ le chlorure de pivaloyle pour former le tert-butyl peroxypivalate, ce perester est par exemple commercialisé sous le nom LUPEROX 11 par la société Arkema ;
▪ le chlorure de 2-ethylhexanoyle pour former le tert-butyl peroxy 2-ethylhexanoate, ce perester est par exemple commercialisé sous le nom LUPEROX 26 par la société Arkema ;
▪ le chlorure de 3,5,5-trimethylhexanoyle pour former le tert-butyl peroxy 3,5,5-trimethylhexanoate, ce perester est par exemple commercialisé sous le nom LUPEROX 270 par la société Arkema ;
▪ l'anhydride acétique pour former le tert-butyl peroxyacetate, ce perester est par exemple commercialisé sous le nom LUPEROX 7 par la société Arkema
▪ le chlorure d'isobutyroyle pour former le tert-butyl peroxyisobutyrate, ce perester est par exemple commercialisé sous le nom LUPEROX 80 par la société Arkema
▪ le chlorure de benzoyle pour former le tert-butyl peroxybenzoate, ce perester est par exemple commercialisé sous le nom LUPEROX P par la société Arkema
▪ le chlorure de néoheptanoyle pour former le tert-butyl peroxyheptanoate, ce perester est par exemple commercialisé sous le nom LUPEROX 701 par la société Arkema.

Dans la fabrication de peroxyacétals ou peracétals, on peut citer l'utilisation de l'hydroperoxyde de tertiobutyle obtenu à partir de matières d'origine renouvelable avec :
▪ la méthyléthylcétone pour former le 2,2-di(tert-butylperoxy)butane, ce produit est par exemple commercialisé sous le nom LUPEROX 220 par la société Arkema,
▪ la 3,3,5-trimethylcyclohexanone pour former le 1,1-di(tert-butylperoxy)-3,3,5-trimethylcyclohexane, ce produit est par exemple commercialisé sous le nom LUPEROX 231 par la société Arkema,
▪ le 3-oxo éthylbutyrate pour former l'éthyl 3,3-di(tert-butylperoxy)butyrate, ce produit est par exemple commercialisé sous le nom LUPEROX 233 par la société Arkema,
▪ la cyclohexanone pour former le 1,1-di (ter-butylperoxy)cyclohexane, ce produit est par exemple commercialisé sous le nom LUPEROX 331 par la société Arkema.

Dans la fabrication de dialkylperoxydes, on peut citer l'utilisation de l'hydroperoxyde de tertiobutyle obtenu à partir de matières d'origine renouvelable avec :
▪ le 4-oxo n-butylvalérate pour former le n-butyl 4,4-di(tert-butylperoxy)valérate, ce produit est par exemple commercialisé sous le nom LUPEROX 230 par la société Arkema,
▪ le cumylperoxyde pour former le tert-butylcumylperoxyde, ce produit est par exemple commercialisé sous le nom LUPEROX 801 par la société Arkema,
▪ du benzène et du propylène pour former le 1,4-bis(tert-butylperoxyisopropyl)benzène, le 1,3(4)-bis(tert-butylperoxyisopropyl)benzène ou le 1,3-bis(tert-butylperoxyisopropyl)benzène, ces produits sont respectivement commercialisés sous les noms LUPEROX 802, LUPEROX F, LUPEROX FM par la société Arkema,
▪ l'isobutène pour former le di-tert-butylperoxde, ce produit est par exemple commercialisé sous le nom LUPEROX DI par la société Arkema.

Dans la fabrication de monoperoxypercarbonates, on peut citer l'utilisation de l'hydroperoxyde de tertiobutyle obtenu à partir de matières d'origine renouvelable avec :
▪ le 2-éthylhexylchloroformate pour former le OO-ter-butyl-O-(2-éthylhéxyl)monoperoxycarbonate, ce produit est par exemple commercialisé sous le nom LUPEROX TBEC par la société Arkema,
▪ l'isopropylchloroformate pour former le OO-ter-butyl-OO-isopropylmonoperoxycarbonate, ce produit est par exemple commercialisé sous le nom LUPEROX TBIC par la société Arkema.

On peut encore citer les utilisations de l'hydroperoxyde de tertiobutyle obtenu à partir de matières d'origine renouvelable en tant qu'initiateur de polymérisation ou encore l'utilisation dudit l'hydroperoxyde de tertiobutyle dans la préparation d'un initiateur de polymérisation.

## Revendications

1. Procédé de fabrication d'hydroperoxyde de tertiobutyle **caractérisé en ce qu'**il comprend les étapes suivantes :
a) fermentation de matières premières renouvelables et, éventuellement purification pour produire un mélange comprenant au moins du butanol ;
b) déshydratation du butanol en butène;
c) conversion du butène en isobutène,
d) réaction avec du peroxyde d'hydrogène de manière à produire de l'hydroperoxyde de tertiobutyle,
e) isolation de l'hydroperoxyde de tertiobutyle.

2. Procédé de fabrication d'hydroperoxyde de tertiobutyle selon la revendication 1 dans lequel l'étape c) est suivie d'une étape d'hydratation de l'isobutène en tertiobutanol, et dans lequel, à l'étape d) le tertiobutanol réagit avec le peroxyde d'hydrogène.

3. Procédé de fabrication d'hydroperoxyde de tertiobutyle selon la revendication 1 ou 2, **caractérisé en ce que** les matières premières renouvelables sont des matières végétales choisies parmi la canne à sucre et la betterave sucrière, l'érable, le palmier-dattier, le palmier à sucre, le sorgho, l'agave américain, le maïs, le blé, l'orge, le sorgho, le seigle, le froment, le riz, la pomme de terre, le manioc, la patate douce, la paille, le bois, le papier, les algues.

4. Procédé de fabrication d'hydroperoxyde de tertiobutyle selon l'une quelconque des revendications précédentes **caractérisé en ce que** l'étape de fermentation a) est effectuée en présence de *Clostridium acetobutylicum* ou un de ses mutants.

5. Procédé de fabrication d'hydroperoxyde de tertiobutyle selon l'une quelconque des revendications précédentes **caractérisé en ce que** l'étape de fermentation a) est suivie d'une étape d'isolation du butanol, de préférence par distillation hétéroazéotropique.

6. Procédé de fabrication d'hydroperoxyde de tertiobutyle selon l'une quelconque des revendications précédentes **caractérisé en ce que** l'étape b) est effectuée en présence d'un acide fort à une température d'environ 120°C.

7. Procédé de fabrication d'hydroperoxyde de tertiobutyle selon l'une quelconque des revendications précédentes **caractérisé en ce que** l'étape c) est effectuée par mise en contact du butène avec un tectométallosilicate ayant une structure cristalline de ferriérite, à une température comprise entre 150 °C et 450 °C, une pression partielle du butène de 0,5 bar et une pression totale comprise entre 0,5 et 25 bar.

8. Procédé de fabrication d'hydroperoxyde de tertiobutyle selon l'une quelconque des revendications précédentes **caractérisé en ce que** l'étape d) s'effectue en phase liquide, en présence d'acide sulfurique, sous agitation continue et vigoureuse, à une température de réaction comprise entre 30°C et 75°C à pression atmosphérique.

9. Procédé de fabrication d'hydroperoxyde de tertiobutyle selon l'une quelconque des revendications précédentes **caractérisé en ce que** l'étape d'hydratation de l'isobutène en tertiobutanol est effectuée en présence d'acide sulfurique dilué, ou en présence de résines échangeuses d'ions acides.

## Patentansprüche

1. Verfahren zur Herstellung von tert.-Butylhydroperoxid, **dadurch gekennzeichnet, dass** es die folgenden Schritte umfasst:
a) Fermentation von erneuerbaren Rohstoffen und gegebenenfalls Aufreinigung, wodurch man zu einer Mischung, die mindestens Butanol umfasst, gelangt;
b) Dehydratation des Butanols zu Buten;
c) Überführung des Butens in Isobuten,
d) Umsetzen mit Wasserstoffperoxid, so dass man zu tert.-Butylhydroperoxid gelangt,
e) Isolieren des tert.-Butylhydroperoxids.

2. Verfahren zur Herstellung von tert.-Butylhydroperoxid, nach Anspruch 1, wobei sich auf Schritt c) ein Schritt der Hydratation des Isobutens zu tert.-Butanol anschließt und wobei in Schritt d) das tert.-Butanol mit dem Wasserstoffperoxid reagiert.

3. Verfahren zur Herstellung von tert.-Butylhydroperoxid, nach Anspruch 1 oder 2, **dadurch gekennzeichnet, dass** es sich bei den erneuerbaren Rohstoffen um pflanzliche Substanzen, ausgewählt aus Zuckerrohr und Zuckerrübe, Ahorn, Dattelpalme, Zuckerpalme, Sorghum, Amerikanische Agave, Mais, Getreide, Gerste, Sorghum, Roggen, Weizen, Reis, Kartoffel, Maniok, Süßkartoffel, Stroh, Holz, Papier und Algen, handelt.

4. Verfahren zur Herstellung von tert.-Butylhydroperoxid, nach einem der vorhergehenden Ansprüche, **dadurch gekennzeichnet, dass** der Fermentationsschritt a) in Gegenwart von *Clostridium acetobutylicum* oder einer seiner Mutanten durchgeführt wird.

5. Verfahren zur Herstellung von tert.-Butylhydroperoxid, nach einem der vorhergehenden Ansprüche, **dadurch gekennzeichnet, dass** sich auf den Fermentationsschritt a) ein Schritt der Isolierung des Butanols, vorzugsweise mittels heteroazeotroper Destillation, anschließt.

6. Verfahren zur Herstellung von tert.-Butylhydroperoxid, nach einem der vorhergehenden Ansprüche, **dadurch gekennzeichnet, dass** Schritt b) in Gegenwart einer starken Säure bei einer Temperatur von ungefähr 120°C durchgeführt wird.

7. Verfahren zur Herstellung von tert.-Butylhydroperoxid, nach einem der vorhergehenden Ansprüche, **dadurch gekennzeichnet, dass** Schritt c) durch Inkontaktbringen des Butens mit einem Tectometallosilicat mit einer Ferrierit-Kristallstruktur bei einer Temperatur zwischen 150°C und 450°C, einem Butenpartialdruck von 0,5 bar und einem Gesamtdruck zwischen 0,5 und 25 bar durchgeführt wird.

8. Verfahren zur Herstellung von tert.-Butylhydroperoxid, nach einem der vorhergehenden Ansprüche, **dadurch gekennzeichnet, dass** Schritt d) in flüssiger Phase in Gegenwart von Schwefelsäure unter kontinuierlichem starkem Rühren bei einer Reaktionstemperatur zwischen 30°C und 75°C bei Atmosphärendruck durchgeführt wird.

9. Verfahren zur Herstellung von tert.-Butylhydroperoxid, nach einem der vorhergehenden Ansprüche, **dadurch gekennzeichnet, dass** der Schritt der Hydratation des Isobutens zu tert.-Butanol in Gegenwart von verdünnter Schwefelsäure oder in Gegenwart von sauren Ionenaustauscherharzen durchgeführt wird.

## Claims

1. A process for producing tert-butyl hydroperoxide, **characterized in that** it comprises the following steps:
a) fermenting renewable starting materials and, optionally, purifying so as to produce a mixture comprising at least butanol;
b) dehydrating the butanol to give butene;
c) converting the butene to give isobutene;
d) reacting with hydrogen peroxide so as to produce tert-butyl hydroperoxide,
e) isolating the tert-butyl hydroperoxide.

2. The process for producing tert-butyl hydroperoxide as claimed in claim 1, in which step c) is followed by a step of hydrating the isobutene to give tert-butanol, and in which, in step d) the tert-butanol reacts with the hydrogen peroxide.

3. The process for producing tert-butyl hydroperoxide as claimed in claim 1 or 2, **characterized in that** the renewable starting materials are plant materials chosen from sugar cane and sugar beet, maple, date palm, sugar palm, sorghum, American agave, corn, wheat, barley, sorghum, rye, soft wheat, rice, potato, cassava, sweet potato, straw, wood, paper and algae.

4. The process for producing tert-butyl hydroperoxide as claimed in any one of the preceding claims, **characterized in that** fermentation step a) is carried out in the presence of *Clostridium acetobutylicum* or a mutant thereof.

5. The process for producing tert-butyl hydroperoxide as claimed in any one of the preceding claims, **characterized in that** fermentation step a) is followed by a step for isolating the butanol, preferably by heteroazeotropic distillation.

6. The process for producing tert-butyl hydroperoxide as claimed in any one of the preceding claims, **characterized in that** in step b) it is carried in the presence of a strong acid at a temperature of approximately 120°C.

7. The process for producing tert-butyl hydroperoxide as claimed in any one of the preceding claims, **characterized in that** step c) is carried out by bringing the butene into contact with a tectometallosilicate which has a ferrierite crystalline structure, at a temperature of between 150°C and 450°C, a butene partial pressure of 0.5 bar and a total pressure of between 0.5 and 25 bar.

8. The process for producing tert-butyl hydroperoxide as claimed in any one of the preceding claims, **characterized in that** step d) is carried out in the liquid phase, in the presence of sulfuric acid, with continuous and vigorous stirring, at a reaction temperature of between 30°C and 75°C at atmospheric pressure.

9. The process for producing tert-butyl hydroperoxide as claimed in claim 2, **characterized in that** the step of hydrating the isobutene to give tert-butanol is carried out in the presence of dilute sulfuric acid, or in the presence of acidic ion exchange resins.
